# EUROPÄISCHE PATENTSCHRIFT

(11) **EP 0 718 282 B1**
(45) Veröffentlichungstag und Bekanntmachung des Hinweises auf die Patenterteilung: **16.05.2001**
(21) Anmeldenummer: 95119231.9
(22) Anmeldetag: 06.12.1995
(51) Int. Cl.: C07C 319/12, C07C 319/28

(54) **Verfahren zur Herstellung von Thioglykolsäurealkylestern**
Process for preparing alkyl esters of thioglycolic acid
Procédé pour la préparation des esters d'alkyle de l'acide thioglycolique

(30) Priorität: 19.12.1994 DE 4445202
(43) Veröffentlichungstag der Anmeldung: 26.06.1996
(73) Patentinhaber: MERCK PATENT GmbH, 64293 Darmstadt (DE)
(72) Erfinder: Schneider, Alexandra, D-63741 Aschaffenburg (DE); Cimbollek, Gerhard, Dr., D-64342 Seeheim (DE); Müller, Bernd, D-64673 Zwingenberg (DE); Heywang, Ulrich, Dr., D-64289 Darmstadt (DE)

(56) Entgegenhaltungen:
- EP-A- 0 421 831
- DATABASE WPI Derwent Publications Ltd., London, GB; AN 80-06540c & JP-A-54 157 504 (ASAHI CHEMICAL IND. KK.) , 12.Dezember 1979
- DATABASE WPI Derwent Publications Ltd., London, GB; AN 92-281683 & JP-A-04 193 851 (NIPPON KAYAKU KK.) , 13.Juli 1992

## Beschreibung

Die Erfindung betrifft ein verbessertes Aufarbeitungsverfahren bei der Herstellung von Thioglycolsäurealkylestern durch Erhitzen eines Reaktionsgemisches, welches im wesentlichen aus Thioglycolsäure, einem Alkohol und einem sauren Katalysator besteht, wobei das gebildete Wasser an einem Wasserabscheider azeotrop abdestilliert wird.

Thioglykolsäureester finden bei der Kunststoffherstellung Verwendung, insbesondere wirken sie der Versprödung von Kunststoffen bei Lichteinfall entgegen.

In der japanischen Offenlegungsschrift JP 54 157504 wird vorgeschlagen, das bei der Veresterung von Carbonsäuren mit einem Alkohol in Gegenwart eines sauren Katalysators anfallende Produktgemisch mit einer wäßrigen alkalischen Lösung zu neutralisieren und die restliche freie Säure an einem stark basischen lonenaustauscher zu absorbieren. Dabei fallen jedoch durch die jeweilige freie Säure belastete Abwässer an.

Nachteilig ist weiterhin, daß bei der Verwendung eines stark basischen lonenaustauschers in Gegenwart von Wasser die Gefahr einer Verseifung des gewünschten Reaktionsprodukts und damit einer erheblichen Ausbeuteminderung besteht.

Aufgabe der vorliegenden Erfindung war es nun ein vereinfachtes Verfahren aufzuzeigen, das es erlaubt, Thioglykolsäurealkylester in höheren Raum-Zeit-Ausbeuten zur Verfügung zu stellen.

Weiterhin war es Aufgabe der Erfindung ein Verfahren aufzuzeigen, bei dem keine mit Thioglykolsäure belasteten Abwässer in die Umwelt gelangen können.

Diese Aufgabe wurde erfindungsgemäß dadurch gelöst, daß das bei der azeotropen Veresterung von Thioglykolsäure mit einem Alkohol erhaltene Produktgemisch nach der Abdestillation von Wasser, gegebenenfalls nach mechanischer Abtrennung des Katalysators, zur Entfernung von Säurespuren über Amberlyst-A21 geleitet wird.

Gegenstand der Erfindung ist somit ein Verfahren zur Herstellung von Thioglycolsäurealkylestern durch Erhitzen eines Reaktionsgemisches, welches im wesentlichen aus Thioglycolsäure, einem Alkohol und einem sauren Katalysator in Form eines sauren lonenaustauschers oder einer thermisch abtrennbaren Säure besteht, wobei das gebildete Wasser an einem Wasserabscheider azeotrop abdestilliert wird, dadurch gekennzeichnet, daß man das erhaltene Produktgemisch, gegebenenfalls nach mechanischer Abtrennung des sauren Katalysators, über Amberlyst A-21 leitet.

Der Begriff "Alkohol" steht für einen geradkettigen oder verzweigten aliphatischen Alkohol mit 2 bis 12, vorzugsweise 5 bis 10 C-Atomen, insbesondere für verzweigte C₆₋₁₀-Alkohole wie zum Beispiel 2-Ethylhexanol.

Als saure Katalysatoren werden solche eingesetzt, die während des Verfahrens entweichen können oder saure Ionenaustauscher, die im Anschluß an das eigentliche Herstellverfahren einfach durch mechanische Methoden vom Produktgemisch abgetrennt werden können und somit nicht zu einer Belastung des basischen lonenaustauschers führen.

Bevorzugte Ausführungsformen des erfindungsgemäßen Verfahrens sind:
- Verfahren, wobei man das erhaltene Produktgemisch destillativ aufreinigt.
- Verfahren, wobei man 2-Ethylhexanol als Alkohol verwendet.
- Verfahren, wobei man die Reaktion bei Temperaturen zwischen 60° und 150° C durchführt.
- Verfahren, wobei man die Reaktion unter reduzierten Druck, vorzugsweise bei Drucken zwischen 300 und 10 mbar, durchführt.
- Verfahren, wobei man 1,0 bis 1,5 mol, insbesondere 1,02 bis 1,10 mol Alkohol bezogen auf 1,0 mol Thioglycolsäure einsetzt.
- Verfahren, wobei man 0,01 bis 0,1 Gewichtsteile an saurem Katalysator bezogen auf ein Gewichtsteil Thioglycolsäure einsetzt.
- Verfahren, wobei man einen sauren lonenaustauscher als sauren Katalysator, insbesondere Amberlyst A-15, einsetzt.

Der Begriff "saurer Ionenaustauscher" steht für einen kommerziell erhältlichen polymeren Kationenaustauscher, z.B. Styrolsulfonsäurepolymerisate wie Lewatit S 100, Zeo-Karb 225, Dowex 50, Amberlite IR 120 oder Amberlyst A-15, Polysulfonsäurekondensate wie Lewatit PN, Zeo-Karb 215 oder 315, m-Phenolkarbonsäureharze wie Lewatit CNO oder Duolite CS 100 oder Polyacrylate wie Permutit C Zeo-Karb 226 oder Amberlite IRC 50.

Besonders bevorzugte saure lonenaustauscher sind makroporöse lonenaustauscher, insbesondere Amberlyst A-15 der Firma Rohm & Haas oder K 2631 der Firma Bayer.

Die Durchführung des erfindungsgemäßen Verfahrens ist an sich einfach. Ein Gemisch bestehend aus der Thioglykolsäure, dem Alkohol und dem sauren Katalysator wird in einem Reaktor vorgelegt. Der Alkohol kann gegebenenfalls auch nach Anlegen eines Unterdruckes hinzugefügt werden. Das Reaktiongemisch wird solange an einem Wasserabscheider zum Sieden erhitzt bis kein Wasser mehr übergeht. Das Produktgemisch wird gegebenenfalls durch Filtration vom sauren Katalysator getrennt und ohne vorheriges Abkühlen kontinuierlich über den mit Amberlyst-A21 beladenen Festbettreaktor geleitet bzw. gepumpt.

Die Überleitung des Reaktionsgemisch erfolgt bis zum Durchbruch des Eduktes, d.h. solange bis im Produktgemisch Thioglykolsäure nachgewiesen wird (z.B. durch Titration mit Tetrabutylammoniumhydroxidlösung zur Bestimmung der freien Säure, Dünnschicht-, HPLC- oder Gaschromatogramm).

Das so erhaltene Rohprodukt wird ohne vorheriges Abkühlen in eine Destillationsapparatur gefüllt und vorzugsweise unter reduziertem Druck, insbesondere bei Drucken zwischen 1 und 20 mbar, insbesondere zwischen 6 und 17 mbar, destilliert.

Der mehrfach eingesetzte lonenaustauscher wird anschließend gewaschen und mit einer Base regeneriert.

Aufgrund des erfindungsgemäßen Verfahrens ist es möglich, Thioglykolsäurealkylester, ein wichtiges Produkt für die Kunststoff erzeugende Industrie, in höheren Raum-Zeit-Ausbeuten und unter Vermeidung von die Umwelt belastenden Abwässern herzustellen. Weiterhin weist das erfindungsgemäße Verfahren eine günstige Energiebilanz auf, da das Produktgemisch zwischen dem eigentlichen Herstellverfahren, der Behandlung mit dem basischen lonenaustauscher und der Destillation nicht auf Raumtemperatur abgekühlt und wieder erwärmt werden muß.

Das nachfolgende Beispiel soll die Erfindung erläutern ohne sie zu begrenzen. Die angegebenen Temperaturen beziehen sich auf Grad Celsius (°C), die angegebenen Drucke auf Millibar (1 mbar = 0,75 Torr).

### Beispiel

### Herstellung von Thioglycolisooctylester

5,6 g Amberlyst A-15 werden in einen Rührwerksapparatur gefüllt. Man fügt gleichzeitig 189,3 g (2,034 mol) Thioglycolsäure und 278,7 g (2,141 mol) 2-Ethylhexanol hinzu. Unter Rühren wird das Gemisch bei einem Druck von 170 mbar solange am Wasserabscheider zum Sieden erhitzt bis kein Wasser mehr übergeht. Nach Abtrennen des gebildeten Wassers wird das Reaktiongemisch auf 85 °C abgekühlt und der lonenaustauscher vom Reaktionsgemisch abgetrennt. Anschließend wird das Produktgemisch über eine Säule mit Amberlyst A-21 geleitet. Der verwendete basische lonenaustauscher wird ohne Regenerierung weiterhin eingesetzt Der Rückstand wird unter reduziertem Druck (8,5 mbar) und einer Kopftemperatur von 105 °C destilliert. Man erhält 340,9 g (82 % d. Th.) des Produktes bei einem Vorlauf von 14,2 g und einem Rückstand von 35,5 g. Es fällt dabei kein mit Thioglycolsäure belastetes Abwasser an.

### Vergleichsbeispiel

Ein Gemisch aus 2,5 g Amberlyst A-15, 71,0 g (0,763 mol) Thioglycolsäure und 109,8 g (0,843 mol) 2-Ethylhexanol wird wie in Beispiel 1 beschrieben am Wasserabscheider erhitzt bis kein Wasser mehr übergeht. Der Rückstand wird bei 25 °C mit 15 ml einer 10 %igen Natriumhydrogencarbonatlösung neutralisiert und mit 15 ml einer 3 %igen Kochsalzlösung gewaschen. Nach Abtrennen der wäßrigen Phase (31,9 g Waschwasser belastet mit Thioglykolsäure) wird der Rückstand unter reduziertem Druck (8,5 mbar) und einer Kopftemperatur von 105 °C destilliert. Man erhält 129,5 g (82 %) des Produktes bei einem Vorlauf von 16,1 g und einem Rückstand von 4,9 g.

## Patentansprüche

1. Verfahren zur Herstellung von Thioglycolsäurealkylestern durch Erhitzen eines Reaktionsgemisches, welches im wesentlichen aus Thioglycolsäure, einem Alkohol und einem sauren Katalysator in Form eines sauren lonenaustauschers oder einer thermisch abtrennbaren Säure besteht, wobei das gebildete Wasser an einem Wasserabscheider azeotrop abdestilliert wird, dadurch gekennzeichnet, daß man das erhaltene Produktgemisch, gegebenenfalls nach mechanischer Abtrennung des sauren Katalysators, über Amberlyst-A21 leitet.

2. Verfahren nach Anspruch 1, dadurch gekennzeichnet, daß man das erhaltene Produktgemisch destillativ aufreinigt.

3. Verfahren nach einem der vorhergehenden Ansprüche, dadurch gekennzeichnet, daß man die 2-Ethylhexanol als Alkohol verwendet.

4. Verfahren nach einem der vorhergehenden Ansprüche, dadurch gekennzeichnet, daß man die Reaktion bei Temperaturen zwischen 60° und 150° C durchführt.

5. Verfahren nach einem der vorhergehenden Ansprüche, dadurch gekennzeichnet, daß man die Reaktion unter reduziertem Druck durchführt.

6. Verfahren nach einem der vorhergehenden Ansprüche, dadurch gekennzeichnet, daß man 1,0 bis 1,5 mol Alkohol bezogen auf 1,0 mol Thioglykolsäure einsetzt.

7. Verfahren nach einem der vorhergehenden Ansprüche, dadurch gekennzeichnet, daß man 0,01 bis 0,1 Gewichtsteile an saurem Katalysator bezogen auf ein Gewichtsteil Thioglykolsäure einsetzt.

8. Verfahren nach einem der vorangehenden Ansprüche, dadurch gekennzeichnet, daß man einen sauren lonenaustauscher als sauren Katalysator einsetzt.

9. Verfahren nach einem der vorhergehenden Ansprüche, dadurch gekennzeichnet, daß man Amberlyst A-15 als sauren lonenaustauscher verwendet.

## Claims

1. Process for the preparation of thioglycolic acid alkyl esters by heating a reaction mixture which essentially comprises thioglycolic acid, an alcohol and an acid catalyst in the form of an acid ion exchanger or a thermally separable acid, the water formed being distilled off azeotropically using a water separator, characterized in that the resulting product mixture is passed over Amberlyst-A21, if appropriate after mechanical removal of the acid catalyst.

2. Process according to Claim 1, characterized in that the resulting product mixture is purified by distillation.

3. Process according to one of the preceding claims, characterized in that the 2-ethylhexanol is used as the alcohol.

4. Process according to one of the preceding claims, characterized in that the reaction is carried out at temperatures between 60° and 150°C.

5. Process according to one of the preceding claims, characterized in that the reaction is carried out under reduced pressure.

6. Process according to one of the preceding claims, characterized in that 1.0 to 1.5 mol of alcohol are employed per 1.0 mol of thioglycolic acid.

7. Process according to one of the preceding claims, characterized in that 0.01 to 0.1 part by weight of acid catalyst is employed per one part by weight of thioglycolic acid.

8. Process according to one of the preceding claims, characterized in that an acid ion exchanger is employed as the acid catalyst.

9. Process according to one of the preceding claims, characterized in that Amberlyst A-15 is used as the acid ion exchanger.

## Revendications

1. Procédé de préparation d'esters alkyliques de l'acide thioglycolique par chauffage d'un mélange réactionnel qui pour l'essentiel est constitué d'acide thioglycolique, d'un alcool et d'un catalyseur acide sous forme d'un échangeur d'ions acide ou d'un acide pouvant être séparé par voie thermique, l'eau qui se forme étant chassée par distillation azéotrope sur un séparateur d'eau, caractérisé en ce qu'on envoie sur de l'Amberlyst A21 le mélange de produits ainsi obtenu, éventuellement après séparation mécanique du catalyseur acide.

2. Procédé selon la revendication 1, caractérisé en ce qu'on purifie par distillation le mélange de produits obtenu.

3. Procédé selon l'une des revendications précédentes, caractérisé en ce qu'on utilise du 2-éthylhexanol en tant qu'alcool.

4. Procédé selon l'une des revendications précédentes, caractérisé en ce qu'on met en oeuvre la réaction à des températures comprises entre 60 et 150°C.

5. Procédé selon l'une des revendications précédentes, caractérisé en ce qu'on met en oeuvre la réaction sous pression réduite.

6. Procédé selon l'une des revendications précédentes, caractérisé en ce qu'on utilise de 1,0 à 1,5 moles d'alcool pour 1,0 mole d'acide thioglycolique.

7. Procédé selon l'une des revendications précédentes, caractérisé en ce qu'on utilise de 0,01 à 0,1 partie en poids de catalyseur acide par partie en poids d'acide thioglycolique.

8. Procédé selon l'une des revendications précédentes, caractérisé en ce qu'on utilise en tant que catalyseur acide un échangeur d'ions acide.

9. Procédé selon l'une des revendications précédentes, caractérisé en ce qu'on utilise de l'Amberlyst A-15 en tant qu'échangeur d'ions acide.
